# EUROPÄISCHE PATENTSCHRIFT

(11) **EP 3 578 130 B1**
(45) Veröffentlichungstag und Bekanntmachung des Hinweises auf die Patenterteilung: **30.09.2020**
(21) Anmeldenummer: 18176349.1
(22) Anmeldetag: 06.06.2018
(51) Int. Cl.: A61B 90/70, A61L 2/07, A61L 2/24

(54) **VERFAHREN ZUM REINIGEN EINES MEDIZINISCHEN HOHLKÖRPERINSTRUMENTS SOWIE VORRICHTUNG ZUM DURCHFÜHREN DES VERFAHRENS**
METHOD FOR CLEANING A MEDICAL HOLLOW INSTRUMENT AND DEVICE FOR IMPLEMENTING THE METHOD
PROCÉDÉ DE NETTOYAGE D'UN INSTRUMENT À CORPS CREUX MÉDICAL AINSI QUE DISPOSITIF DE MISE EN OEUVRE DUDIT PROCÉDÉ

(43) Veröffentlichungstag der Anmeldung: 11.12.2019
(73) Patentinhaber: Elma Schmidbauer GmbH, 78224 Singen (DE)
(72) Erfinder: Richter, Andreas, 78224 Singen (DE)
(74) Vertreter: Patentanwälte Behrmann Wagner PartG mbB

(56) Entgegenhaltungen:
- WO-A1-2011/134970
- DE-A1- 2 932 565
- DE-A1- 19 860 290
- DE-A1-102005 026 487
- DE-A1-102010 028 340
- US-A1- 2005 047 957
- US-B1- 6 447 718
- US-B1- 6 733 727

## Beschreibung

Die Erfindung betrifft ein Verfahren zum Reinigen eines medizinischen Hohlkörperinstruments nach dem Oberbegriff des Anspruchs 1 sowie eine Vorrichtung zum Reinigen eines medizinischen Hohlkörperinstruments nach dem Oberbegriff des Anspruchs 8.

Medizinische Hohlkörperinstrumente sind zumindest abschnittsweise rohrförmig ausgebildet und weisen einen Durchmesser auf, welcher erheblich kleiner als ihre Länge ist. So kann deren Holkörperinnendurchmesser bzw. das Schaftrohr beispielsweise 0,5mm bis 12mm betragen. Ein Beispiel für ein solches Hohlkörperinstrument ist ein Trokar. Derartige Hohlkörperinstrumente werden zumeist in der minimalinvasiven Chirurgie genutzt, um beispielsweise Gewebeproben durch den Hohlraum des Instruments zu führen.

An derartige medizinische bzw. chirurgische Instrumente sind sehr hohe Hygieneanforderungen gestellt, welche beispielsweise durch entsprechende Grenzwerte und Richtlinien der Deutschen Gesellschaft für Krankenhaushygiene oder der Deutschen Gesellschaft für Sterilgutversorgung belegt sind. Bisher bekannte Aufbereitungsverfahren sind zumeist mehrstufig aufgebaut, wobei sie zumindest eine Vorreinigung, eine Hauptreinigung sowie einen Desinfektionsschritt, eine Funktionsprüfung sowie eine abschließende Sterilisierung aufweisen.

Für die Vorreinigung eines Hohlkörperinstruments ist die Verwendung eines Ultraschallreinigungsgerätes bekannt. Hierbei werden mittels zumindest eines Ultraschallwandlers Ultraschallwellen in eine Flüssigkeit eingebracht, in welcher sich das Hohlkörperinstrument befindet.

Zudem ist zur Vorreinigung eines Hohlkörperinstruments die Nutzung von Dampf bekannt, welcher in das Hohlkörperinstrument eingeblasen wird. Das Einblasen des Dampfes in das Hohlkörperinstrument erfolgt derart, dass eine Ruhephase, in welcher kein Dampf eingeblasen wird, auf eine relativ langanhaltende Dampfeinbringphase folgt.

Eine derart durchgeführte Vorreinigung führt jedoch dazu, dass Verunreinigungen an dem Hohlkörperinstrument zurückbleiben können, welche in einem anschließenden Sterilisierungsschritt zwar zu "toter Materie" umgewandelt werden, jedoch weiterhin eine nicht akzeptable Kontamination des Hohlkörperinstruments darstellen.

Sowohl die Nutzung von Ultraschall, wie auch die Verwendung von Dampf durch Reinigungsvorrichtungen reinigen ein Hohlkörperinstrument nicht in ausreichendem Maße und zufriedenstellender Regelmäßigkeit, sodass vorgegebene Hygienegrenzwerte nicht sicher eingehalten werden können.

So definieren beispielsweise Akzeptanzkriterien für die Beurteilung der Reinigungsleistung Richtwerte, wonach ein Hohlkörperinstrument bis 4mm Innendurchmesser des Schaftrohres weniger als 75µg und ein Hohlkörperinstrument über 4mm Innendurchmesser des Schaftrohres weniger als 100µg Verunreinigung bzw. Protein pro Prüfkörper als Rinderserumalbumin (BSA) aufweisen sollte. Derartige Grenzwerte werden von den bekannten Vorrichtungen bei der Nutzung in der Vorreinigung jedoch nicht sicher und regelmäßig erreicht.

Aus der DE 29 32 565 ist ein Verfahren und eine Vorrichtung zur Ultraschallreinigung und Sterilisation von Gegenständen in einem Druckbehälter bekannt. Eine Reinigung erfolgt in einer Reinigungsflüssigkeit bei Unterdruckatmosphäre und gleichzeitiger Ultraschallbeaufschlagung.

Die WO 2011/134970 A1 offenbart ein Verfahren zur maschinellen Reinigung und Desinfektion von Gegenständen. Hierzu werden die Gegenstände zunächst in Wasser mir Ultraschall gereinigt worauf das Wasser bis auf ein Restvolumen entfernt und sodann verdampft wird zur Desinfektion der Gegenstände.

Aus der DE 10 2010 028 340 A1 ist ebenfalls eine Vorrichtung zur Reinigung und Desinfektion von Gegenständen bekannt, wobei die Gegenstände zur Reinigung in ein flüssiges Medium eingelegt und mit in Leitungskomponenten der Vorrichtung eingekoppelten Ultraschallwellen beaufschlag werden.

Die DE 198 60 290 A1 beschreibt ein Sterilisationsverfahren sowie einen Dampfsterilisator, in welchem zu reinigende Gegenstände mittels Dampf vorgereinigt und anschließend getrocknet werden.

Aus der US 2005/0047957 A1 ist ein Autoklav bekannt sowie ein Betriebsverfahren zur Sterilisation von Gegenständen.

Aufgabe der Erfindung ist es somit, ein Verfahren zum Reinigen eines medizinischen Hohlkörperinstruments sowie eine Vorrichtung zum Durchführen des Verfahrens zu schaffen, welche die hohen Hygieneanforderungen erfüllen.

Diese Aufgabe ist sowohl durch das Verfahren mit den Merkmalen des Anspruchs 1 wie auch durch die Vorrichtung mit den Merkmalen des Anspruchs 8 gelöst.

Erfindungsgemäß wird ein Verfahren zum Reinigen eines medizinischen Hohlkörperinstruments vorgeschlagen, welches die folgenden Schritte umfasst:
- Bereitstellen einer Vorrichtung, umfassend ein Wannenelement, welches einen mit einer Flüssigkeit füllbaren Füllraum definiert, zumindest einen Ultraschallwandler, welcher über einen zugeordneten Emissionsabschnitt des Wannenelements Ultraschallwellen in das Wannenelement einkoppelt, einen Träger, welcher zum Halten des Hohlkörperinstruments in der Flüssigkeit des Wannenelements ausgebildet ist, eine Steuerung zum Steuern des Reinigungsvorganges, und eine Dampferzeugungsvorrichtung zur Erzeugung von Dampf und zum wahlweisen Einleiten des Dampfes über eine Zuleitung in das Hohlkörperinstrument;
- Füllen des Wannenelements bis zu einer definierten Füllhöhe mit Flüssigkeit des Wannenelements;
- Anordnen zumindest eines zu reinigenden Hohlkörperinstruments an dem Träger;
- Einbringen des Trägers in die Flüssigkeit des Wannenelements, sodass das Hohlkörperinstrument vollständig von der Flüssigkeit umgeben ist;
- Einstrahlen eines Dampfes in das Hohlkörperinstrument mittels der Dampferzeugungsvorrichtung für eine Dauer von 0,5s bis 3s, wobei die Dampftemperatur höher als die Temperatur der Flüssigkeit des Wannenelements ist;
- Ultraschallbeschallen des Wannenelements bzw. der Flüssigkeit des Wannenelements mittels des Ultraschallwandlers für eine Dauer von 15s bis 60s, wobei das Einstrahlen und das Ultraschallbeschallen einen Zyklus definieren; und
- mehrfaches Wiederholen des Zyklus für einen definierte Verfahrensdauer.

Unter Dampf wird verdampfte Flüssigkeit verstanden, welche sich zur Ausbildung eines Dampfschlags eignet. Unter Dampfschlag wird die Folge der implosionsartigen Kondensation von Dampfbläschen in oder an einer gegenüber dem Dampf kälteren Flüssigkeit bzw. der Flüssigkeit des Wannenelements verstanden.

Die Verfahrensschritte müssen nicht zwingend in der beschriebenen Reihenfolge durchgeführt werden. So ist es beispielsweise für die Erfindung unerheblich, ob das Hohlkörperinstrument zuerst an dem Träger angeordnet und dann mit dem Träger in die Flüssigkeit eingebracht wird oder anders herum. Es ist ebenfalls unerheblich zu welchem Zeitpunkt die Flüssigkeit in das Wannenelement eingebracht wird, solange dies zeitlich vor der Durchführung des Zyklus geschieht. Die Verfahrensschritte sollten jedoch in einer Reihenfolge geschehen, bei welcher sich die erfindungsgemäße Wirkung einstellt.

Die vorteilhafte Wirkung des Verfahrens ergibt sich insbesondere aus der Kombination des Einstrahlens eines Dampfes und der Ultraschallbeschallung. Die Temperatur der Flüssigkeit des Wannenelements bzw. die Temperatur der in das Wannenelement eingefüllten Flüssigkeit kann 30 °C bis 40 °C betragen. Die Temperatur des Dampfes kann mindestens 100 °C, insbesondere 120 °C betragen.

Das Einstrahlen des Dampfes, welcher sich bei Kontakt mit der Flüssigkeit des Wannenelements schlagartig abkühlt, führt zu einem Dampfschlag. Der entstehende Dampfschlag führt dazu, dass das das Hohlkörperinstrument umgebende Medium bzw. die Flüssigkeit in das Hohlkörperinstrument mit hohen Kräften eingesaugt oder eingeschossen wird. Hierbei entstehen an der Grenzfläche zwischen dem Dampf und der Flüssigkeit enorm große Scherkräfte, welche u.a. zum Ablösen von Verunreinigungen führen können.

Zudem wird das mit Flüssigkeit gefüllte Hohlkörperinstrument ultraschallbeschallt. Ein weiterer erfinderischer Effekt ergibt sich daraus, dass das Einstrahlen relativ kurz andauert und die Ultraschallbeschallung verhältnismäßig lange andauert, wobei das Zeitverhältnis zwischen Einstrahlen und Beschallen im Bereich von 1:120 bis 1:5 liegt.

Es ist denkbar, dass das Einstrahlen für eine Dauer von 2s erfolgt und/oder das Ultraschallbeschallen für eine Dauer von 30s, vorzugsweise 28s erfolgt. Bei einer derartigen Kombination dieser beiden Zeitspannen kann ein Zyklus aus Einstrahlen und Beschallen innerhalb von 30s erfolgen, wobei hier ein Zeitverhältnis von Einstrahlen zu Beschallen von 1:14 ausgebildet ist. Es ist zudem oder alternativ denkbar, dass eine Verfahrensdauer 360s beträgt und/oder der Zyklus zwölfmal wiederholt wird. Hierbei kann somit eine vollständige Vorreinigung eines Hohlkörperinstruments in lediglich 6min erfolgen.

Bei einer weiteren bevorzugten Ausführungsform des Verfahrens nach der Erfindung wird der Dampf mit einem einzigen Stoß bzw. Puls konstanten Drucks zwischen 4 bar bis 8 bar während eines Einstrahlens eines Zyklus eingestrahlt. Mit einem solchen Druckbereich ist das Verfahren an Hohlkörperinstrumente unterschiedlicher Abmessungen anpassbar.

Hierbei ist hinsichtlich des gewählten Drucks insbesondere von Interesse, wie viel Luft und/oder Flüssigkeit aus dem Hohlkörperinstrument durch ein Einstrahlen herausgedrängt wird und/oder wie viel Flüssigkeit durch einen entstandenen Dampfschlag in das Hohlkörperinstrument eingeschossen wird.

Bei einer weiteren bevorzugten Ausführungsform des Verfahrens nach der Erfindung wird das Einstrahlen derart ausgeführt, dass das Hohlkörperinstrument vollständig mit Dampf gefüllt ist. Mit anderen Worten wird die in dem Hohlkörperinstrument vorhandene Luft oder die in dem Hohlkörperinstrument vorhandene Flüssigkeit vollständig aus diesem herausgepresst. Das führt u.a. dazu, dass sich die Grenzfläche zwischen dem Dampf und der Flüssigkeit bzw. der Luft zumindest an den Außenwänden des Hohlkörperinstruments befindet oder zumindest in dessen Bereich. Dadurch entstehen die Scherkräfte dort, wo die Verunreinigung vorhanden ist.

Das Einstrahlen kann derart erfolgen, dass ein entstehender Dampfschlag und das Einschießen der Flüssigkeit mit so hoher Geschwindigkeit erfolgen, dass die Flüssigkeit bis zu einem Rückschlagventil der Vorrichtung reicht.

Zur gleichzeitigen Reinigung mehrerer Hohlkörperinstrumente kann ein Träger verwendet werden, welcher mehrere Trageplätze aufweist. An jedem dieser Trageplätze oder Trägermittel kann somit ein Hohlkörperinstrument angeordnet werden und die Steuerung kann derart ausgebildet sein, dass jedes einzelne Hohlkörperinstrument gleichzeitig mit oder unabhängig von den anderen Hohlkörperinstrumenten und Trägermitteln mit dem oben genannten Zyklus beaufschlagt wird.

Es ist beispielsweise denkbar, dass ein Hohlkörperinstrument unter Dampfeinstrahlung steht, während gleichzeitig die übrigen Hohlkörperinstrumente ultraschallbeschallt werden. Nach dem Dampfschlag und dem Füllen mit Flüssigkeit des ersten Hohlkörperinstruments findet eine Ultraschallbeschallung auch dieses ersten Hohlkörperinstruments statt, woraufhin ein bisher ultraschallbeschalltes zweites Hohlkörperinstrument eine Dampfeinstrahlung erfahren kann. Gleichzeitig werden die übrigen Hohlkörperinstrumente ultraschallbeschallt. Nach dem Einstrahlen und dem entstandenen Dampfschlag ist das zweite Hohlkörperinstrument mit Flüssigkeit gefüllt und wird ultraschallbeschallt, woraufhin ein drittes Hohlkörperinstrument das Einstrahlen von Dampf erfahren kann. Mit anderen Worten ist denkbar, dass das Wannenelement durchgehend mit Ultraschallwellen beschallt wird und die einzelnen Hohlkörperinstrumente nacheinander oder gleichzeitig ein Dampfeinstrahlen erfahren.

Erfindungsgemäß ist zudem eine Vorrichtung zum Reinigen zumindest eines Hohlkörpergutes vorgeschlagen. Die Vorrichtung umfasst ein Wannenelement, welches einen mit einer Flüssigkeit füllbaren Füllraum definiert, zumindest einen Ultraschallwandler, welcher über einen zugeordneten Emissionsabschnitt des Wannenelements Ultraschallwellen in das Wannenelement einkoppelt, einen Träger, welcher zum Halten des zumindest einen Hohlkörperinstruments in der Flüssigkeit ausgebildet ist, eine Steuerung zum Steuern des Reinigungsvorganges, und eine Dampferzeugungsvorrichtung zur Erzeugung von Dampf und zum wahlweisen Einleiten von Dampf über eine Zuleitung in das Hohlkörperinstrument.

Die Steuerung ist zum Durchführen des folgenden Reinigungsprogramms ausgebildet:
- Einstrahlen eines Dampfes in das zumindest eine Hohlkörperinstrument mittels der Dampferzeugungsvorrichtung für eine Dauer von 0,5s bis 3s,
- Ultraschallbeschallen des Wannenelements bzw. der Flüssigkeit des Wannenelements mittels des Ultraschallwandlers für eine Dauer von 15s bis 60s, wobei das Einstrahlen und das Ultraschallbeschallen einen Zyklus definieren; und
- mehrfaches Wiederholen des Zyklus für einen definierte Verfahrensdauer.

Mittels der Steuerung ist es somit in vorteilhafter Weise möglich, das erfindungsgemäße Verfahren nach einem der Ansprüche 1 bis 7 durchzuführen. Dadurch ergeben sich auch die oben genannten Vorteile, welche ebenfalls die Probleme des Standes der Technik überwinden und insbesondere zu einer hochqualitativen Vorreinigung von medizinischen Hohlkörperinstrumenten führen.

Bei einer bevorzugten Ausführungsform der Vorrichtung nach der Erfindung weist der Träger mehrere Trägerplätze bzw. Trägermittel zum Halten von je einem Hohlkörperinstrument auf. Dadurch können zugleich mehrere Hohlkörperinstrumente, vorzugsweise innerhalb eines einzigen Wannenelements, gereinigt werden.

Bei einer weiteren bevorzugten Ausführungsform der Vorrichtung nach der Erfindung ist die Steuerung derart ausgebildet, dass sie die Zyklen auf die Hohlkörperinstrumente unabhängig voneinander anwendet. Dadurch ist es möglich, die Reinigungsdauer für mehrere Hohlkörperinstrumente zu reduzieren, da nämlich eine Ultraschallreinigung von Hohlkörperinstrumenten in der Zeit erfolgen kann, in welcher ein einziges Hohlkörperinstrument eine Dampfeinstrahlung erfährt.

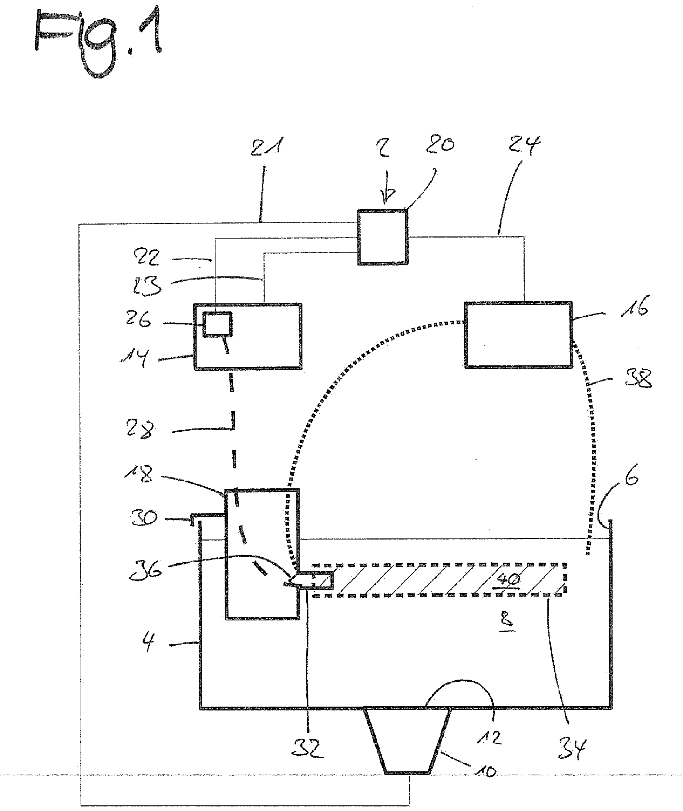

Gemäß einer weiteren Ausführungsform der Vorrichtung nach der Erfindung ist die Dampferzeugungsvorrichtung ausgebildet zur Einstellung einer Dampftemperatur. Die Dampferzeugungsvorrichtung dient zur Erwärmung und Überhitzung von einer Initialflüssigkeit, insbesondere über 100 °C. Dadurch entsteht Dampf, welcher eine Dampftemperatur von beispielsweise 140 °C bis 180 °C aufweisen kann. Die Vorrichtung kann zusätzlich oder alternativ eine Dampfkammer zum Halten des erzeugten Dampfes haben. Es ist auch denkbar, dass die Dampferzeugungsvorrichtung ein Ventil, vorzugsweise ein Magnetventil, aufweist, mittels welchem ein Flussweg zwischen der Dampferzeugungsvorrichtung und dem Hohlkörperinstrument wahlweise verschließbar oder freigebbar ist. Die Dampferzeugungsvorrichtung kann so ausgebildet sein, dass die Erwärmung des Dampfes bzw. die Dampftemperatur in Abhängigkeit von der Temperatur der Flüssigkeit des Wannenelements gewählt wird, um einen geeigneten Dampfschlag zu erzeugen, welcher eine größtmögliche Reinigungswirkung entfaltet. Zudem kann eine Pumpenvorrichtung vorgesehen sein, welche zum wahlweisen Pumpen von Flüssigkeit in das Hohlkörperinstrument dient. Dabei kann die Flüssigkeit entweder die in dem Wannenelement vorhandene Flüssigkeit sein oder aber eine zusätzliche Flüssigkeit. Durch eine derartige Pumpe ist ein Spülen des Hohlkörperinstruments möglich. Weitere Vorteile, Merkmale und Einzelheiten der Erfindung ergeben sich aus der Beschreibung bevorzugter Ausführungsbeispiele sowie anhand der einzigen Figur, welche eine schematische Blockdarstellung einer Vorrichtung zum Reinigen eines medizinischen Hohlkörperinstruments zeigt.

Zur Vermeidung von Wiederholungen sollen vorrichtungsgemäß offenbarte Merkmale auch als verfahrensgemäß offenbart gelten.

Ebenso sollen verfahrensgemäß offenbarte Merkmale auch als vorrichtungsgemäß offenbart gelten.

Die Vorrichtung 2 umfasst ein Wannenelement 4, welches einen Füllraum 6 definiert, der zumindest teilweise mit einer Flüssigkeit 8 gefüllt ist. Die Flüssigkeit 8 des Wannenelements kann eine Flüssigkeitstemperatur von 30 °C bis 40 °C haben. An dem Wannenelement 4 ist ein Ultraschallwandler 10 angeordnet, welcher über einen zugeordneten Emissionsabschnitt 12 des Wannenelements 4 Ultraschallwellen in das Wannenelement 4 einkoppelt. Die Vorrichtung 2 umfasst zudem eine Dampferzeugungsvorrichtung 14, eine Pumpeneinrichtung 16, einen Träger 18 sowie eine Steuerung 20.

Die Steuerung 20 ist mittels einer ersten Datenleitung 21 signaltechnisch an dem Ultraschallwandler 10 angeschlossen, mittels einer zweiten Datenleitung 22 signaltechnisch an eine Dampfkammer 26 angeschlossen, mittels einer dritten Datenleitung 23 an die Dampferzeugungsvorrichtung 14 angeschlossen und mittels einer vierten Datenleitung 24 an die Pumpeneinrichtung 16 angeschlossen. Die Datenleitungen 21 bis 24 dienen zum Übertragen von Steuersignalen von der Steuerung 20 an den Ultraschallwandler 10, die Dampfkammer 26, die Dampferzeugungsvorrichtung 14 und/oder die Pumpeneinrichtung 16. Es ist denkbar, dass die Datenleitungen 21 bis 24 auch zur Übertragung von Daten, wie beispielsweise gemessene Sensordaten, der einzelnen Elemente 10, 14, 24 und/oder 26 hin zur Steuerung 20 dienen.

Die Dampferzeugungsvorrichtung 14 umfasst die Dampfkammer 26 und ist über eine Dampftransportzuleitung 28 mit dem Träger 18 verbunden. Die Dampferzeugungsvorrichtung 14 erwärmt und überhitzt eine Initialflüssigkeit zu Erzeugung von Dampf derart, dass die Dampftemperatur des Dampfes 40 oberhalb der Flüssigkeitstemperatur der Flüssigkeit 8 liegt. Die Initialflüssigkeit kann beispielsweise durch Überhitzung eine Initialflüssigkeitstemperatur von 140 °C bis 180 °C und/oder der Dampf eine Dampftemperatur haben. Der erzeugte Dampf wird in der Dampfkammer 26 vorgehalten und kann über die Dampftransportzuleitung 28 wahlweise zum Träger 18 transportiert werden. In dem Flussweg zwischen der Dampferzeugungsvorrichtung 14 und dem Träger 18 ist ein Magnetventil zum wahlweisen Verschließen oder Freigeben des Weges angeordnet. Bei Entnahme des Dampfes aus der Dampfkammer kann der Dampf aufgrund Abkühlung durch Ausdehnung eine Temperatur von 120 °C haben.

Der Träger 18 ist mittels eines geeigneten Befestigungsmittels 30 an dem Wannenelement 4 fixierbar, um beispielsweise ein Aufschwimmen zu verhindern und um eine Prozessposition zu definieren. Der Träger 18 weist einen Trägerplatz bzw. ein Trägermittel 32 auf, um ein medizinisches Hohlkörperinstrument 34 aufzunehmen. Das Hohlkörperinstrument 34 ist derart an dem als Trägerplatz 32 dienenden Trägermittel fixiert, dass es dauerhaft oder zumindest für die Verfahrensdauer vollständig von Flüssigkeit 8 umgeben ist und ein Dampfeinstrahlen die Fixierung nicht löst. Das Trägermittel 32 ist über eine Ventilanordnung 36 mit der Dampftransportzuleitung 28 verbunden. Obgleich lediglich ein einziges Trägermittel 32 abgebildet ist, kann der Träger 18 mehrere gleiche oder unterschiedliche Trägermittel 32 für jeweils ein Hohlkörperinstrument 34 aufweisen.

Ebenfalls mit der Ventilanordnung 36 ist eine Flüssigkeitszuleitung 38 der Pumpeneinrichtung 16 verbunden. Mittels der Pumpeneinrichtung 16 ist die Flüssigkeit 8 wahlweise in das Hohlkörperinstrument 34 via der Ventilanordnung 36 einpumpbar.

Das medizinische Hohlkörperinstrument 34 kann beispielsweise einen Innendurchmesser von 0,5mm bis zu 12mm und eine Länge von beispielsweise 100mm bis 500mm haben.

Ein von der Steuerung 20 gesteuertes Reinigungsverfahren und/oder Aufbereitungsverfahren kann, wie nachfolgend erläutert ablaufen.

Das auf dem Trägermittel 32 fixierte Hohlkörperinstrument 34 ist zu Beginn mit Luft gefüllt und kann durch den in der Flüssigkeit 8 anliegenden Flüssigkeitsumgebungsdruck mit dieser langsam volllaufen. Vor oder während des Reinigungsvorganges kann die Flüssigkeitstemperatur mittels eines nicht gezeigten Temperaturmessmittels gemessen und die Messdaten an die Steuerung 20 übertragbar sein. In jedem Fall ist die Steuerung 20 dazu ausgebildet, die Dampferzeugungsvorrichtung 14 derart anzusteuern, dass der erzeugte Dampf 40 eine Temperatur hat, welche oberhalb der Flüssigkeitstemperatur liegt; hierfür dient die Dampferzeugungsvorrichtung 14. In das Hohlkörperinstrument 34 wird der erwärmte Dampf 40 für eine Dauer von 0,5s bis 3s pulsartig eingestrahlt. Dieses Einstrahlen führt dazu, dass die anfangs in dem Hohlkörperinstrument 34 vorhandene Luft und die darin unter Umständen vorhandene Flüssigkeit zumindest teilweise, vorzugsweise ganz, aus dem Hohlkörperinstrument 34 ausgepresst wird. Der Unterschied zwischen Dampftemperatur und Flüssigkeitstemperatur führt dazu, dass eine implosionsartige Kondensation der Dampfbläschen bzw. ein Dampfschlag erfolgt. Dieser führt dazu, dass die das Hohlkörperinstrument 34 umgebende Flüssigkeit 8 schlagartig in das Hohlkörperinstrument 34 eingesaugt wird. Durch die an der Grenzfläche zwischen Dampf 40 und Flüssigkeit 8 entstehenden Scherkräfte kann Schmutz und Verunreinigung von dem Hohlkörperinstrument 34 gelöst werden. Anschließend oder aber auch während des Einstrahlens von Dampf 40 kann das Hohlkörperinstrument 34 mittels des Ultraschallwandlers 10 ultraschallbestrahlt werden. Eine Ultraschallbestrahlung zwischen zwei Dampfeinstrahlungen erfolgt für eine Dauer von 15s bis 60s. Das Einstrahlen von Dampf 40 und das Beschallen mittels Ultraschall definieren einen Zyklus. Dieser Zyklus ist für eine definierte Verfahrensdauer von 360s ausführbar und/oder der Zyklus ist zwölfmal wiederholbar.

Das Einstrahlen des Dampfes 40 kann derart erfolgen, dass die durch den Dampfschlag einschießende Flüssigkeit 8 ein Rückschlagventil der Ventilanordnung 36 erreicht.

### Bezugszeichenliste

- 2: Vorrichtung
- 4: Wannenelement
- 6: Füllraum
- 8: Flüssigkeit
- 10: Ultraschallwandler
- 12: Emissionsabschnitt
- 14: Dampferzeugungsvorrichtung
- 16: Pumpeneinrichtung
- 18: Träger
- 20: Steuerung
- 21: 1. Datenleitung
- 22: 2. Datenleitung
- 23: 3. Datenleitung
- 24: 4. Datenleitung
- 26: Dampfkammer
- 28: Dampfzuleitung
- 30: Befestigungsmittel
- 32: Trägermittel
- 34: Hohlkörperinstrument
- 36: Ventilanordnung
- 38: Flüssigkeitszuleitung

## Patentansprüche

1. Verfahren zum Reinigen eines medizinischen Hohlkörperinstruments (34), umfassend die folgenden Schritte:
- Bereitstellen einer Vorrichtung, umfassend ein Wannenelement (4), welches einen mit einer Flüssigkeit (8) füllbaren Füllraum (6) definiert, zumindest einen Ultraschallwandler (10), welcher über einen zugeordneten Emissionsabschnitt (12) des Wannenelements (4) Ultraschallwellen in das Wannenelement (4) einkoppelt, einen Träger (18), welcher zum Halten des Hohlkörperinstruments (34) in der Flüssigkeit (8) des Wannenelements (4) ausgebildet ist, eine Steuerung (20) zum Steuern des Reinigungsvorganges, und eine Dampferzeugungsvorrichtung (14) zur Erzeugung von Dampf und zum wahlweisen Einleiten des Dampfes über eine Zuleitung (28) in das Hohlkörperinstrument (34);
- Füllen des Wannenelements (4) bis zu einer definierten Füllhöhe mit Flüssigkeit (8) des Wannenelements (4);
- Anordnen zumindest eines zu reinigenden Hohlkörperinstruments (34) an dem Träger (18);
- Einbringen des Trägers (18) in die Flüssigkeit (8) des Wannenelements (4), so dass das Hohlkörperinstrument (34) vollständig von der Flüssigkeit (8) umgeben ist;
**gekennzeichnet durch**
- Einstrahlen eines Dampfes in das Hohlkörperinstrument (34) mittels der Dampferzeugungsvorrichtung (14) für eine Dauer von 0,5s bis 3s, wobei die Dampftemperatur höher als die Temperatur der Flüssigkeit des Wannenelements (4) ist;
- Ultraschallbeschallen des Wannenelements (4) bzw. der Flüssigkeit (8) des Wannenelements (4) mittels des Ultraschallwandlers (10) für eine Dauer von 15s bis 60s, wobei das Einstrahlen und das Ultraschallbeschallen einen Zyklus definieren; und
- mehrfaches Wiederholen des Zyklus für einen definierte Verfahrensdauer.

2. Verfahren nach Anspruch 1, wobei
das Einstrahlen für eine Dauer von 2s erfolgt.

3. Verfahren nach Anspruch 1 oder 2, wobei
das Ultraschallbeschallen für eine Dauer von 30s, vorzugsweise 28s erfolgt.

4. Verfahren nach einem der vorangehenden Ansprüche, wobei
die Verfahrensdauer 360s beträgt und/oder der Zyklus zwölfmal wiederholt wird.

5. Verfahren nach einem der vorangehenden Ansprüche, wobei der Dampf mit einem konstanten Druck zwischen 4 bar bis 8 bar während eines Einstrahlens eingestrahlt wird.

6. Verfahren nach einem der vorangehenden Ansprüche, wobei das Einstrahlen so ausgeführt wird, dass das Hohlkörperinstrument (34) vollständig mit dem Dampf gefüllt ist.

7. Verfahren nach einem der vorangehenden Ansprüche, wobei ein Träger (18) verwendet wird, welcher mehrere Trägerplätze zum Halten von je einem Hohlkörperinstrument (34) aufweist, und/oder die Steuerung (20) derart ausgebildet ist, dass sie die Zyklen auf die Hohlkörperinstrumente (34) unabhängig voneinander anwendet.

8. Vorrichtung zum Reinigen zumindest eines medizinischen Hohlkörperinstruments (34), umfassend
ein Wannenelement (4), welches einen mit einer Flüssigkeit (8) füllbaren Füllraum (6) definiert,
zumindest einen Ultraschallwandler (10), welcher über einen zugeordneten Emissionsabschnitt (12) des Wannenelements (4) Ultraschallwellen in das Wannenelement (4) einkoppelt,
einen Träger (18), welcher zum Halten des zumindest einen Hohlkörperinstruments (34) in der Flüssigkeit (8) ausgebildet ist, eine Steuerung (20) zum Steuern des Reinigungsvorganges, und eine Dampferzeugungsvorrichtung (14) zur Erzeugung von Dampf und zum wahlweisen Einleiten des Dampfes und zum wahlweisen Einleiten des Dampfes über eine Zuleitung (28) in das zumindest eine Hohlkörperinstrument (34),
**dadurch gekennzeichnet, dass**
die Steuerung zum Durchführen des folgenden Reinigungsprogramms ausgebildet ist:
- Einstrahlen eines Dampfes in das zumindest eine Hohlkörperinstrument (34) mittels der Dampferzeugungsvorrichtung (14) für eine Dauer von 0,5s bis 3s,
- Ultraschallbeschallen des Wannenelements (4) bzw. der Flüssigkeit (8) des Wannenelements (4) mittels des Ultraschallwandlers (10) für eine Dauer von 15s bis 60s, wobei das Einstrahlen und das Ultraschallbeschallen einen Zyklus definieren; und
- mehrfaches Wiederholen des Zyklus für einen definierte Verfahrensdauer.

9. Vorrichtung nach Anspruch 8, wobei
der Träger mehrere Trägerplätze zum Halten von je einem Hohlkörperinstrument (34) aufweist.

10. Vorrichtung nach Anspruch 8 oder 9, wobei die Steuerung (20) derart ausgebildet ist, dass sie die Zyklen auf die Hohlkörperinstrumente (34) unabhängig voneinander anwendet.

11. Vorrichtung nach einem der vorangehenden Ansprüche, wobei die Dampferzeugungsvorrichtung (14) eine Dampfkammer (26) aufweist.

12. Vorrichtung nach einem der vorangehenden Ansprüche, wobei eine Pumpeneinrichtung (16) vorgesehen ist zum wahlweisen Pumpen von Flüssigkeit (8) in das Hohlkörperinstrument (34).

## Claims

1. A method for cleaning a medical hollow instrument (34) comprising the following steps:
- providing a device comprising a tub element (4) defining a filling chamber (6) to be filled with a liquid (8), at least one ultrasound transducer (10) coupling ultrasonic waves into the tub element (4) via an assigned emission section (12) of the tub element (4), a support (18) designed for holding the hollow instrument (34) in the liquid (8) of the tub element (4), a controller (20) for controlling the cleaning process, and a steam generation device (14) for generating steam and for the optional introduction of the steam into the hollow instrument (34) via a supply line (28);
- filling the tub element (4) with liquid (8) of the tub element (4) up to a defined filling level;
- disposing at least one hollow instrument (34) to be cleaned on the support (18);
- introducing the support (18) into the liquid (8) of the tub element (4), the hollow instrument (34) thus being entirely surrounded by the liquid (8),
**characterized by**
- injecting steam into the hollow instrument (34) by means of the steam generation device (14) for a period of 0.5 s to 3 s, the steam temperature being higher than the temperature of the liquid of the tub element (4);
- ultrasonicating the tub element (4) or the liquid (8) of the tub element (4) by means of the ultrasound transducer (10) for a period of 15 s to 60 s, the injection and the ultrasonication defining a cycle; and
- repeating the cycle multiple times for a defined process duration.

2. The method according to claim 1,
wherein the injection is performed for a period of 2 s.

3. The method according to claim 1 or 2,
wherein the ultrasonication is performed for a period of 30 s, preferably 28 s.

4. The method according to any one of the preceding claims,
wherein the process duration is 360 s and/or the cycle is repeated twelve times.

5. The method according to any one of the preceding claims,
wherein the steam is injected at a constant pressure between 4 bar to 8 bar during the injection.

6. The method according to any one of the preceding claims,
wherein the injection is realized in such a manner that the hollow instrument (34) is entirely filled with the steam.

7. The method according to any one of the preceding claims,
wherein a support (18) is used which has several support spaces for supporting one hollow instrument (34) each, and/or the controller (20) is realized in such a manner that said controller (20) applies the cycles to the hollow instruments (34) independently of one another.

8. A device for cleaning at least one medical hollow instrument (34), the device comprising
a tub element (4) defining a filling chamber (6) to be filled with a liquid (8),
at least one ultrasound transducer (10) coupling ultrasonic waves into the tub element (4) via an assigned emission section (12) of the tub element (4),
a support (18) designed for holding the at least one hollow instrument (34) in the liquid (8),
a controller (20) for controlling the cleaning process, and
a steam generation device (14) for generating steam and for the optional introduction of the steam into the at least one hollow instrument (34) via a supply line (28),
**characterized in that**
the controller is realized for performing the following cleaning program:
- injecting steam into the at least one hollow instrument (34) by means of the steam generation device (14) for a period of 0.5 s to 3 s,
- ultrasonicating the tub element (4) or of the liquid (8) of the tub element (4) by means of the ultrasound transducer (10) for a period of 15 s to 60 s, the injection and the ultrasonication defining a cycle; and
- repeating the cycle multiple times for a defined process duration.

9. The device according to claim 8,
wherein the support has several support spaces for supporting one hollow instrument (34) each.

10. The device according to claim 8 or 9,
wherein the controller (20) is realized in such a manner that said controller (20) applies the cycles to the hollow instruments (34) independently of one another.

11. The device according to any one of the preceding claims,
wherein the steam generation device (14) has a steam chamber (26).

12. The device according to any one of the preceding claims,
wherein a pump device (16) is provided for the optional pumping of liquid (8) into the hollow instrument (34).

## Revendications

1. Procédé de nettoyage d'un instrument creux (34) médical comprenant les étapes suivantes :
- fournir un dispositif comprenant un élément de cuve (4) qui définit une chambre de remplissage (6) qui peut être remplie d'un liquide (8), au moins un transducteur ultrasonore (10) qui couple des ondes ultrasonores dans l'élément de cuve (4) via une partie d'émission (12) assignée de l'élément de cuve (4), un support (18) destiné à retenir l'instrument creux (34) dans le liquide (8) de l'élément de cuve (4), un contrôleur (20) destiné à contrôler l'opération de nettoyage, et un dispositif de génération de vapeur (14) destiné à la génération de vapeur et à l'introduction sélective de la vapeur dans l'instrument creux (34) via une conduite d'alimentation (28) ;
- remplir l'élément de cuve (4) de liquide (8) de l'élément de cuve (4) jusqu'à une hauteur de remplissage définie ;
- disposer au moins un instrument creux (34) à nettoyer sur le support (18) ;
- introduire le support (18) dans le liquide (8) de l'élément de cuve (4), de sorte que l'instrument creux (34) est entouré entièrement par le liquide (8) ;
**caractérisé par** les étapes consistant à
- injecter une vapeur dans l'instrument creux (34) au moyen du dispositif de génération de vapeur (14) pour une durée de 0,5 s à 3 s, la température de vapeur étant supérieure à la température du liquide de l'élément de cuve (4) ;
- appliquer de l'ultrason à l'élément de cuve (4) ou au liquide (8) de l'élément de cuve (4) au moyen du transducteur ultrasonore (10) pour une durée de 15 s à 60 s, l'injection et l'application d'ultrason définissant un cycle ; et
- répéter le cycle plusieurs fois pour une durée de procédé définie.

2. Procédé selon la revendication 1,
dans lequel l'injection est réalisée pour une durée de 2 s.

3. Procédé selon la revendication 1 ou la revendication 2,
dans lequel l'application d'ultrason est réalisée pour une durée de 30 s, de préférence 28 s.

4. Procédé selon l'une quelconque des revendications précédentes,
dans lequel la durée de procédé est 360 s et/ou le cycle est répété douze fois.

5. Procédé selon l'une quelconque des revendications précédentes,
dans lequel la vapeur est injectée à une pression constante entre 4 bar à 8 bar pendant une injection.

6. Procédé selon l'une quelconque des revendications précédentes,
dans lequel l'injection est réalisée de telle manière que l'instrument creux (34) est rempli entièrement de la vapeur.

7. Procédé selon l'une quelconque des revendications précédentes,
dans lequel un support (18) est utilisé qui a plusieurs places de support destinée à retenir chacune un instrument creux (34), et/ou que le contrôleur (20) est réalisé de telle manière qu'il applique les cycles aux instruments creux (34) indépendamment l'un de l'autre.

8. Dispositif de nettoyage d'au moins un instrument creux (34) médical, le dispositif comprenant
un élément de cuve (4) qui définit une chambre de remplissage (6) qui peut être remplie d'un liquide (8),
au moins un transducteur ultrasonore (10) qui couple des ondes ultrasonores dans l'élément de cuve (4) via une partie d'émission (12) assignée de l'élément de cuve (4),
un support (18) destiné à retenir l'au moins un instrument creux (34) dans le liquide (8),
un contrôleur (20) destiné à contrôler l'opération de nettoyage, et
un dispositif de génération de vapeur (14) destiné à la génération de vapeur et à l'introduction sélective de la vapeur dans l'au moins un instrument creux (34) via une conduite d'alimentation (28), **caractérisé en ce que**
le contrôleur est destiné à effectuer le programme de nettoyage suivant :
- injecter une vapeur dans l'au moins un instrument creux (34) au moyen du dispositif de génération de vapeur (14) pour une durée de 0,5 s à 3 s ;
- appliquer de l'ultrason à l'élément de cuve (4) ou au liquide (8) de l'élément de cuve (4) au moyen du transducteur ultrasonore (10) pour une durée de 15 s à 60 s, l'injection et l'application d'ultrason définissant un cycle ; et
- répéter le cycle plusieurs fois pour une durée de procédé définie.

9. Dispositif selon la revendication 8,
dans lequel le support a plusieurs places de support destinées à retenir chacune un instrument creux (34).

10. Dispositif selon la revendication 8 ou la revendication 9,
dans lequel le contrôleur (20) est réalisé de telle manière qu'il applique les cycles aux instruments creux (34) indépendamment l'un de l'autre.

11. Dispositif selon l'une quelconque des revendications précédentes,
dans lequel le dispositif de génération de vapeur (14) a une chambre de vapeur (26).

12. Dispositif selon l'une quelconque des revendications précédentes,
dans lequel un dispositif de pompe (16) est prévu pour le pompage sélectif de liquide (8) dans l'instrument creux (34).
